(19) Europäisches Patentamt / European Patent Office / Office européen des brevets

(11) **EP 4 151 740 A1**

(12) **EUROPEAN PATENT APPLICATION**

(43) Date of publication:
**22.03.2023 Bulletin 2023/12**

(21) Application number: **22196092.5**

(22) Date of filing: **16.09.2022**

(51) International Patent Classification (IPC):
**C12P 7/46** (2006.01)   **C12P 7/6409** (2022.01)
**C12P 7/44** (2006.01)   **C12N 15/01** (2006.01)
**C12R 1/74** (2006.01)

(52) Cooperative Patent Classification (CPC):
**C12P 7/46; C12N 15/01; C12P 7/44; C12P 7/6409;**
C12R 2001/74

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.09.2021 IN 202121042329**

(71) Applicant: **Indian Oil Corporation Limited
Bandra (East) Maharashtra, Mumbai 400 051 (IN)**

(72) Inventors:
• **SAHOO, Prakash Chandra**
  **121007 Haryana (IN)**

• **KUMAR, Manoj**
  **121007 Haryana (IN)**
• **SINGH, Dheer**
  **121007 Haryana (IN)**
• **PURI, Suresh Kumar**
  **121007 Haryana (IN)**
• **RAMAKUMAR, Sankara Sri Venkata**
  **121007 Haryana (IN)**

(74) Representative: **Pons
Glorieta Rubén Darío 4
28010 Madrid (ES)**

Remarks:
The complete document including Reference
Table(s) and the Sequence Listing(s) can be
downloaded from the EPO website

(54) **A BIOASSISTED PROCESS FOR SELECTIVE CONVERSION OF ALKANE RICH REFINERY STREAM**

(57)  The present invention relates to a process for valorization of low-cost alkane rich feedstock. More specifically, the present invention relates to the selective conversion of alkane rich kerosene to value-added products like mono/dicarboxylic acid, fatty acids and biosurfactants using mutant yeast strain and a heterogenous nano-catalyst. The present invention also provides a mutant yeast strain for selective conversion of alkane rich refinery stream from a substrate containing hydrocarbons. The mutant yeast strain of the present invention is able to consume the sulfur content in the feed and results in the desulfurization of the alkane rich feedstock.

**Description**

**FIELD OF THE INVENTION**

The present invention relates to a process for valorization of low-cost alkane rich feedstock. More specifically, the present invention relates to the selective conversion of alkane rich kerosene to value-added products like mono/dicarboxylic acid, fatty acids and biosurfactants using mutant yeast strain and a heterogenous nano-catalyst.

**BACKGROUND OF THE INVENTION**

Valorization of alkane rich streams and surplus/low-cost hydrocarbon feedstock is an important method to enhance their cost competitiveness. For instance, low-cost hydrocarbon streams like kerosene is an inevitable product obtained from the fractional distillation of petroleum between 150 °C and 275°C with a density of 0.78-0.81 g/cm$^3$. It is a mixture of hydrocarbons with number of carbon atoms ranging from 6 to 16. The major components are branched and straight chain alkanes and naphthene's (cycloalkanes), which normally account for at least 70% by volume. Kerosene has been used as a fuel for the jet engine, domestic lighting, and cooking in many countries. However, due to high sulfur content, emission of pollutants like CO, $CO_2$, and carbon black the use of kerosene consumption has been reduced in many countries including India. Therefore, the surplus kerosene produced in petroleum refineries needs to be used and valorized to avoid storage issues. Conversion of kerosene to chemicals that have a high market value is one of the options for its valorization.

There are few prior arts which describes the conversion of an alkane to valuable chemicals by both chemical and biological method. Chemically the alkane stream from the refinery is treated in several ways including cracking, isomerization and reforming to form valuable chemicals and additives. However, these processes are highly energy intensive, involves multistep conversion processes and also results in unwanted and hazardous byproducts.

WO2006064131A1 discloses a biological process for producing dicarboxylic acids (DCA) from long hydrocarbon chains using a mutant strain.

Kitamoto et.al (Biotechnology Letters 2001, 23, 1709-1714,) described a process for conversion of n-Alkanes ranging from C12 to C18 into glycolipid biosurfactants, mannosyl erythritol lipids (MEL), by resting cells of *Pseudozyma* (Candida) Antarctica.

CN102191187A discloses a *candida lipolytica* strain and a method for preparing long chain dicarboxylic acid by using the *candida lipolytica* strain, in a culture medium in which C10-C18 n-alkane is used as a substrate.

US 2004/0146999 A1 describes a bioprocess for converting aliphatic compounds, of the form $CH_3 (CH_2) nCH_3$ Where n=4 to 20, to mono-terminal and di-terminal carboxylates using genetically engineered organisms.

US 2013/0267012 A1 provides a method for the biosynthesis of dicarboxylic acid ranging in length from C3 to C26. The host cell can be further modified to increase fatty acid production or export of the desired fatty acid derived compound, and/or decrease fatty acid storage or metabolism.

WO2007077568A1 discovered strains which are capable of producing a long-chain dicarboxylic acid by culturing microorganisms to produce a long-chain dicarboxylic acid in a liquid medium containing a straight-chain saturated hydrocarbon as substrate.

Das *et al.,* 2019 relates to isolation and identification of yeast strain capable of degrading indeno (1,2,3-cd) pyrene (InP). The document describes biodegradation of InP in presence of iron nanoparticles and produced biosurfactant in the growth medium. However, the document describes production of sophorolipid biosurfactant by *C. tropicalis* NN4 which enhanced the degradation of indeno (1,2,3-cd) pyrene (InP) in the growth medium.

Mandal *et al.,* 2018 discloses degradation of benzo[a]pyrene (BaP) using a yeast consortium YC01 in presence of zinc oxide nanoparticles and produced biosurfactant in the growth medium. The document also describes various process parameters viz. pH (A: 5.0-9.0), temperature (B: 10-50° C), shaking sped (C: 110-150 rpm), inoculum dosage (D: 1-5%) and ZnO nanoparticle concentration (E: 1-3 g/L) for the process optimization.

WO 2010/068904 A2 discloses a process of making linear dicarboxylic acid of $C_n$ chain length. The document also discloses a catalyst which comprises an oxide, molybdenum, and one or more active metals selected from the group consisting of nickel, cobalt, and mixtures thereof and further, the catalyst is in sulfided form. Further, the document particularly describes that the linear alkane of $C_n$ chain length, or portion thereof, is fermented with a transformed *Candida maltosa* strain SW84/87.2 identified as ATCC 74430.

US 10,604,775 B2 describes a method for producing dioic acids from a substrate containing hydrocarbons or fatty acids selected from the group consisting of mutant strain (*Candida infanticola* LC-DA01; KCTC13099BP), transformant strain (*Candida infanticola* KCTC13103BP, KCTC13104BP, KCTC13105BP, or KCTC13106BP) or a combination thereof.

US 8,383,373 B2 discloses a process of producing long-chain dicarboxylic acid by culturing microorganisms belonging to *Candida vini, Candida entamophila, Candida blankii* and *Pichia farinosa.* The document also discloses production of long-chain dicarboxylic acid in presence of a liquid medium containing a straight-chain saturated hydrocarbon (tridecane) as substrate.

Shioo and R. Uohio *et al.*,1971 describes microbial production of long chain dicarboxylic acid (DC) from n-alkane. The document describes screening of strains isolated from oil-soaked soil around the oil refinery and production of dicarboxylic acids from yeasts belonging to genus *Candida* and *Pichia.*

Several processes for bioconversion of alkane to dicarboxylic acids have been described in the prior arts, however the drawbacks of the above-said bioprocess are that the feedstock usually consists of commercial grade straight chain alkanes, which are expensive and difficult to avail. Moreover, the presence of impurities in the feedstock hampers the biosynthesis and selectivity. Therefore, these methods described in the prior art are not suitable for the bioconversion feedstock, in particular alkane rich petroleum-rich feedstock having mixed alkane moieties along with pollutants like sulfur. Further, the processes reported in the prior art results in formation of a single product.

Thus, there is a need to develop mutant strains which are able to withstand harsh feed conditions and can selectively convert various alkanes to value added products. Further, to develop a process for conversion of alkane rich refinery stream into valuable products.

## SUMMARY OF THE INVENTION

In an aspect of the present invention, there is provided a bio-assisted process for selective conversion of alkane rich refinery stream, the process comprising:

(i) growing a mutant yeast strain in 2% malt media at a temperature of 37°C with rotation at 200rpm till OD of the media reaches 2-4;

(ii) centrifuging the media of step (i) to obtain wet microbial cells;

(iii) adding the centrifuged wet microbial cells of step (ii) to a heterogenous nanocatalyst in a culture media, followed by intermittent feeding of a stabilizer in concentration ranging from 50-100 ppm;

(iv) incubating the media of step (iii) with 1-25% stream in a reactor at a temperature ranging from 35-37°C for a duration of 1-2hours;

(v) sparging oxygen gas to the reactor to obtain a product, wherein the product is selected from mono and dicarboxylic acid, fatty acids and biosurfactants.

In an embodiment of the present invention, there is provided a process wherein the mutant yeast strain is selected from the group consisting of *Candida vini, Candida entamophila, Candida blankii* and *Pichia Farinosa, Candida tropicalis (Castellani) Berkhout* (ATCC 750).

In another embodiment of the present invention, there is provided a process wherein the mutant yeast strain is *Candida tropicalis* (Castellani) Berkhout (ATCC 750).

In an embodiment of the present invention, there is provided a process wherein the obtained product is dicarboxylic acid.

In yet another embodiment of the present invention, there is provided a process wherein dicarboxylic acid is undecanoic acid.

In still another embodiment of the present invention, there is provided a process wherein the alkane rich refinery stream comprises sulphur in the range of 5 ppm to 25000 ppm.

In yet another embodiment of the present invention, there is provided a process wherein the alkane rich refinery stream comprises hydrocarbons having carbon atoms ranging from 6 to 16.

In an embodiment of the present invention, there is provided a process wherein the oxygen gas sparged in the reactor contains 20-100% $O_2$ along with nitrogen and atmospheric air.

In another embodiment of the present invention, there is provided a process wherein the heterogenous nano catalyst comprises:

(i) oxides of molybdenum, vanadium, antimony, niobium;

(ii) at least one element selected from the group consisting of lithium, titanium, tin, germanium, zirconium, hafnium; and

(iii) optionally at least one lanthanide selected from the group consisting of lanthanum, praseodymium, neodymium, samarium, europium, gadolinium, dysprosium, holmium, erbium, thulium, ytterbium and lutetium.

In yet another embodiment of the present invention, there is provided a process wherein the heterogenous nano catalyst is iron doped cerium (III) carbonate.

In still another embodiment of the present invention, there is provided a process wherein the stabilizer is selected from long chain alkylamine or N-based alkylamines and derivatives, N-[2-[(2-Aminoethyl) amino] ethyl]-9-octadecenamide, n-Benzalkonium chloride (BAC), $C_nH(2n+1)$-COO$(CH_2CH_2O)12CH_3$, polyoxyethylene alkyl ether, n-alkyl trimethyl ammonium surfactant, potassium alkanoate, dodecylpyridinium bromide, octylglucoside, Sodium dodecyl sulfate, trans-Cinnamaldehyde, Sodium bis-(2-ethylhexyl)-sulfosuccinate, cetylpyridinium chloride, Primary alcohol ethoxylate, polyoxyethylene nonyl phenyl ether, polyethylene glycol esters, linoleate and dodecyl amine.

In another embodiment of the present invention, there is provided a process wherein the obtained product is separated by column chromatography and un-reacted alkane is circulated back to reactor for fermentation.

In another aspect of the present invention, there is provided a mutant yeast strain for selective conversion of alkane rich refinery stream from a substrate containing hydrocarbons selected from the group consisting of *Candida vini*, *Candida entamophila*, *Candida blankii* and *Pichia Farinosa*, *Candida tropicalis* (Castellani) Berkhout.

These and other features, aspects, and advantages of the present subject matter will be better understood with reference to the following description. This summary is provided to introduce a selection of concepts in a simplified form.

## BRIEF DESCRIPTION OF DRAWINGS

These and other features, aspects, and advantages of the present invention will become better understood when the following detailed description is read with reference to the accompanying drawings in which like characters represent like parts throughout the drawings wherein:

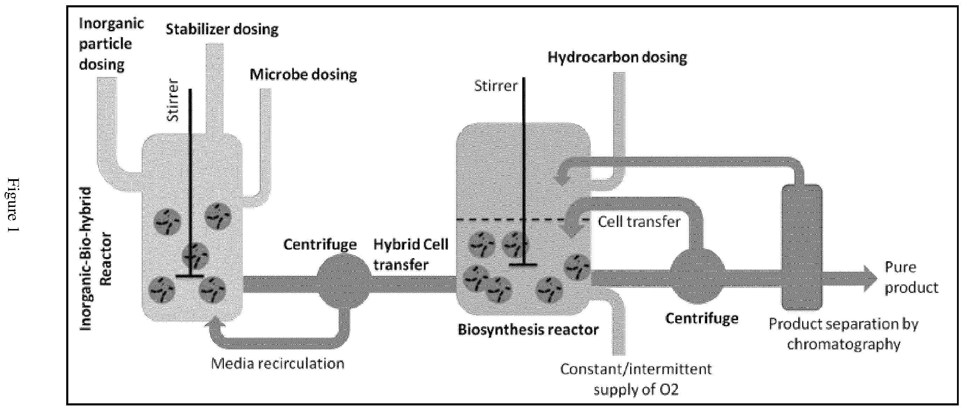

**Figure 1** illustrates continuous reactor system for conversion of alkane rich refinery feedstock.

**Figure 2** illustrates (a) Product formation with different biocatalyst. The product formation was observed at 25% $O_2$ condition and analyzed after 6hours of biotransformation; (b) the selectivity of product at a different $O_2$ concentration; (c) the concentration of total sulfur in the feedstock at different intervals of time.

## DETAILED DESCRIPTION OF THE INVENTION

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

*Definition*:

For the purposes of this invention, the following terms will have the meaning as specified therein:
The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to" "including" and "including but not limited to" are used interchangeably.

The present invention relates to a process for conversion of alkane rich hydrocarbons like kerosene to di/monocarboxylic acids, fatty acids and biosurfactants and other valuable chemicals with high selectivity in presence of a heterogenous nano-catalyst and oxygen. Specifically, the present invention discloses a bioassisted process for selective conversion of alkane rich petroleum refinery feed. In the process, the conversion of alkanes is mediated in the presence of mutant yeast strain selected from the group consisting of *Candida vini*, *Candida entamophila*, *Candida blankii* and *Pichia Farinosa*, *Candida tropicalis* (Castellani) Berkhout. The alkane rich feed stock used consists of low-cost hydrocarbon streams from petroleum refinery containing carbon atoms ranging from 6 to 16. Further, the sulfur content in the hydrocarbon streams may range from 5 ppm to 25000 ppm. In the process of the present invention, initially enriched mutant of *Candida tropicalis* (Castellani) Berkhout (ATCC 750) was selected and grown overnight in 2% malt media at 37°C with rotation at 200rpm, when cultured on alkanes or fatty acids as the carbon. Simultaneously, in a separate process, a heterogenous nano catalyst i.e., iron- doped $CeO_2$ was prepared. Subsequently, in the process, after the OD of the enriched culture reached from 2-4, it was centrifuged and transferred to a medium containing prepared nanocatalyst in a suitable culture medium. This was followed by addition of a stabilizer and finally, the culture medium was inoculated at 37°C for 2 days. Different concentration of oxygen was sparged and the obtained samples were collected at different time points for product quantification and identification by gas chromatography (GC).

The process of the present invention can operate in both continuous and batch mode. Further, the process works at ambient conditions and is selective to any specific product depending on the controlled conditions.

Thus, according to one aspect of the present invention, there is provided a bio-assisted process for selective conversion of alkane rich refinery stream, the process comprising:

(i) growing a mutant yeast strain in 2% malt media at a temperature of 37°C with rotation at 200rpm till OD of the media reaches 2-4;

(ii) centrifuging the media of step (i) to obtain wet microbial cells;

(iii) adding the centrifuged wet microbial cells of step (ii) to a heterogenous nanocatalyst in a culture media, followed by intermittent feeding of a stabilizer in concentration ranging from 50-100 ppm;

(iv) incubating the media of step (iii) with 1-25% stream in a reactor at a temperature ranging from 35-37°C for a duration of 1-2hours;

(v) sparging oxygen gas to the reactor to obtain a product, wherein the product is selected from mono and dicarboxylic acid, fatty acids and biosurfactants.

In an embodiment of the present invention, there is provided a process wherein the mutant yeast strain is selected from the group consisting of *Candida vini, Candida entamophila, Candida blankii and Pichia Farinosa, Candida tropicalis (Castellani) Berkhout.* In a preferred embodiment of the present invention, the mutant yeast strain is *Candida tropicalis* (Castellani) Berkhout (ATCC 750).

The mutant yeast strains described herein are tolerant sulfur content from 5-25000 ppm in the hydrocarbon stream and can grow in presence of a heterogeneous nano-catalyst and are able to form a stable cluster on its surface. Further, the yeast strains described herein can withstand a pH of 3-10.

The mutant yeast strains are cultivated in a culture medium containing carbon source as alkane rich refinery feed stand and other conventional nutrients, such as a nitrogen source and an inorganic salt. Examples of suitable nitrogen sources include organic and inorganic nitrogen-containing compounds, such as peptone, urea, ammonium phosphate, ammonium chloride, ammonium sulfate and ammonium nitrate. Examples of the inorganic salts include phosphates, sulfates, and hydrochlorides of sodium, potassium, magnesium, iron, nickel, and zinc, such as $KH_2PO4$, $K_2HPO4$, $Na_2HPO_4.12H_2O$, $MgSO_47H_2O$, $FeSO_47H_2O$, $ZnSO_4JH_2O$, and NaCl. Moreover, other nutrients, such as yeast extract, meat extract, and D-biotin, are added to the medium for assisting the growth of the yeast.

In another embodiment of the present invention, there is provided a process wherein the alkane rich refinery stream comprises sulphur in the range of 5 ppm to 25000 ppm.

In yet another embodiment of the present invention, there is provided a process wherein the alkane rich refinery stream comprises hydrocarbons having carbon atoms ranging from 6 to 16. Particularly, the alkane rich feed stock described herein comprises of the low-cost alkane rich stream like kerosene or any other stream containing major components as branched and straight chain alkanes and cycloalkanes, which normally account for at least 70% by volume.

In an embodiment of the present invention, there is provided a process wherein the heterogenous nano catalyst comprises:

(i) oxides of molybdenum, vanadium, antimony, niobium;

(ii) at least one element selected from the group consisting of lithium, titanium, tin, germanium, zirconium, hafnium; and

(iii) optionally at least one lanthanide selected from the group consisting of lanthanum, praseodymium, neodymium, samarium, europium, gadolinium, dysprosium, holmium, erbium, thulium, ytterbium and lutetium.

The heterogeneous nano-catalyst consists of bio-compatible transition metal/bimetallic catalyst capable of activating $O_2$ at ambient conditions. The yeast strains are grown on a mixed metal oxide nanoparticle selected from at least one of: (i) heteropolyacids, (ii) aluminas, (iii) zirconias, (iv) titania, (v) zeolites, and (vi) transition metal series and combinations thereof.

In a preferred embodiment of the present invention, there is provided a process wherein the heterogenous nano catalyst is iron doped cerium (III) carbonate. The iron- doped $CeC_2$ was prepared by the following procedure: Typically, 0.02 M solution of Ce (III) nitrate was prepared in 250mL distilled water followed by addition of 0.005 M $FeCl_2$. Similarly, 0.03 M of $K_2CO_3$ solution was prepared by dissolving required amount of $K_2CO_3$ in 250 mL of distilled water. An aqueous solution of iron-containing Ce (III) nitrate (50 mL) and potassium carbonate (20 mL) was added drop by drop to a well-stirred water (100 mL) to precipitate a white-brown precursor, namely iron doped cerium (III) carbonate. The neutral pH was maintained during the precipitation method. Resulting iron doped $CeO_2$ were dried at 65°C for 2 hours, cooled to room temperature. Then, the product was aged at 220°C for 2.5 hours without any washing and purification and finally calcined at 600°C for 3 hours.

In still another embodiment of the present invention, there is provided a process wherein the oxygen gas sparged in the reactor contains 20-100% oxygen along with nitrogen and atmospheric air. The oxygen can be passed through the culture medium through in any suitable device forming the fine dispersion of gas result in an increase in contact area. Further, the oxygen gas can be sparged in micro-bubble or nano-bubble size.

In an embodiment of the present invention, there is provided a process wherein the supply of oxygen gas is continuous or intermittent.

In another embodiment of the present invention, the percentage of oxygen can vary from 5-100%.

In yet another embodiment of the present invention, there is provided a process wherein the stabilizer is selected from long chain alkylamine or N-based alkylamines and derivatives, N-[2-[(2-Aminoethyl) amino] ethyl]-9-octadecenamide, n-Benzalkonium chloride (BAC), $CnH(2n+1)-COO(CH_2CH_2O)12CH_3$, polyoxyethylene alkyl ether, n-alkyl trimethyl ammonium surfactant, potassium alkanoate, dodecylpyridinium bromide, octylglucoside, Sodium dodecyl sulfate, trans-Cinnamaldehyde, Sodium bis-(2-ethylhexyl)-sulfosuccinate, cetylpyridinium chloride, Primary alcohol ethoxylate, polyoxyethylene nonyl phenyl ether, polyethylene glycol esters, Linoleate and dodecyl amine. In a preferred embodiment of the present invention, the stabilizer is polyoxyethylene nonyl phenyl ether. The concentration of the stabilizer used in the present invention can be varied from 0-100 ppm and can be added in continuous or intermittent basis. In a preferred embodiment of the present invention, the stabilizer is added in the concentration of 50-100ppm.

In an embodiment of the present invention, there is provided a process wherein the obtained product is selected from mono-dicarboxylic acids, dicarboxylic acids, fatty acids and biosurfactants.

In another embodiment of the present invention, there is provided a process wherein the product is dicarboxylic acid. In a preferred embodiment of the present invention, dicarboxylic acid is undecanoic acid.

In still another embodiment of the present invention, there is provided a process wherein the obtained product is separated by column chromatography and un-reacted alkane is circulated back to reactor for fermentation. In a preferred embodiment of the present invention, the obtained product can be isolated by chromatography, membrane separation or solvent extraction.

The obtained product can have a selectivity of at least 90%or higher. The product may be glycolipid biosurfactants, mannosylerythritol lipids, dicarboxylic acid, and mono carboxylic acid. Alternatively, the long chain dicarboxylic acids have a purity of at least 95% or higher. In some cases, the long chain dicarboxylic acid is a saturated or unsaturated straight chain dicarboxylic acid having 9 to 18 carbon atoms, with a carboxyl group at two ends of the chain.

In an embodiment of the present invention, the fermentation carried out in the reactor is submerged (stirred tank reactor, bubble column, air- lift, membrane, packed bed and fluidized bed bioreactors) and solid-state fermentation processes (horizontal drum, tray-type, packed bed bioreactor and bench-scale bioreactors) for the production of important biomolecules for industrial applications.

In another aspect of the present invention, there is provided a mutant yeast strain for selective conversion of alkane rich refinery stream from a substrate containing hydrocarbons selected from the group consisting of *Candida vini, Candida entamophila, Candida blankii and Pichia Farinosa, Candida tropicalis (Castellani) Berkhout.*

The novel bio-inorganic hybrid process described herein can convert any alkane rich streams with high sulfur content from 5-25000 ppm to valuable chemicals like mono and dicarboxylic acid, fatty acids and biosurfactants. Further, the process can convert any stream containing straight, branched and cyclo-alkanes to valuable products. The heterogenous nano catalyst used in the process, enhances the feed conversion and selectivity by assisting the partial oxidation of the feed in presence of oxygen. Also, the stabilizer described herein increases the adhesion of microbes on the surface of a heterogeneous inorganic catalyst. In addition, the mutant yeast strains can consume the sulfur content in the feed and results in the desulfurization of the alkane rich feedstock. Moreover, the product selectivity can be tailored by intermittent controlled feeding of the substrate and $O_2$ in an optimized ration. The feed conversion started after 3-4hours of the inoculation and the obtained product is not limited to DCA and results in the formation of monocarboxylic acid, biosurfactant, and other fatty acids.

Although the subject matter has been described in considerable detail with reference to certain preferred embodiments thereof, other embodiments are possible.

**EXAMPLES**

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods, the exemplary methods, devices and materials are described herein. It is to be understood that this disclosure is not limited to particular methods, and experimental conditions described, as such methods and conditions may vary.

**Example 1: Development of mutant of *Candida tropicalis* (Castellani) Berkhout (ATCC 750) and its characterization:**

**(a) Characteristics of Wild strain (*Candida tropicalis* (Castellani) Berkhout (ATCC 750)**

The wild strain *Candida tropicalis* (Castellani) Berkhout (ATCC 750) belongs to fungi classification and was obtained from American Type Culture Collection (ATCC). The typical growth media for the wild strain includes yeast extract (3.0g/L), malt extract (3.0g/L), dextrose (10.0 g/L), peptone (5.0g/L), agar (if required) 20.0 g/L, pH 6.2 +/- 0.2. The typical temperature for growth was between 24°C to 26°C under aerobic conditions.

**(b) Mutagenesis protocol of *Candida tropicalis* (Castellani) Berkhout (ATCC 750)**

The wild strain of *Candida tropicalis* (Castellani) Berkhout (ATCC 750) was inoculated into fresh medium (yeast extract (3.0g/L), malt extract (3.0g/L), dextrose (10.0 g/L), peptone (5.0g/L), agar (if required) 20.0 g/L, pH 6.2 +/- 0.2). Cells at optical density 1.8 at 600nm were centrifuged at 8000 rpm for 10 minutes, washed twice with 0.1 M sodium phosphate buffer (pH 6.0), and were resuspended in a tube with 5 ml sterile saline water of 0.9% (wt/vol) at a concentration of $3.0 \times 10^{11}$ cells ml$^{-1}$. Further, 400$\mu$l of the above cell suspension was placed in a sterile petri dish and exposed to UV rays (235 nm) at a distance of 20cm. After a duration of 5 minutes, the irradiated cell samples were collected, diluted $10^7$-fold, and 100-$\mu$l samples of each of the diluted suspensions were plated on YPD medium. After about 48 hours of cultivation at 38°C, all the individual colonies on each plate with approximately 23 individual colonies were inoculated on slants. The colonies from the slants were tested for the cell capacity to survive in 25% of kerosene in 100 ml YPD broth, and the wild strain was used as a control culture under the same conditions except the wild culture was not irradiated. Based on the highest OD at 600 nm (OD as a measure of survival capacity with 25% of kerosene), one strain with highest survival capacity was selected.

The obtained strain was further subjected to N-methyl-N'-nitro-N-nitrosoguanidine (NTG) treatment. For NTG-treatment, a Log growth phase of strain in YPD media were prepared (OD: 1.3 at 600 nm). 9ml of which was added to 1ml of a sterile solution of NTG (3 mg/ml NTG in phosphate buffer; solution freshly prepared 1hour before use). The samples were shaken at 38°C for 30 minutes and immediately centrifuged for 10 minutes at 8,000 rpm, and the supernatant was decanted. The cells were washed three times with sterile water and resuspended in 10ml of sterile phosphate buffer (pH 7.2). All the experimental samples were serially diluted with sterile water and plated on YPD plates. The YPD plates were grown for 72hour at 38°C. 37 morphologically different colonies were selected and investigated for DCA production using hexadecane (25%) as feed in YPD medium. Finally, the mutants that produces highest DCA yield within 72 hours were considered positive mutants because of its higher DCA production capacity than that of the wild strain.

Table 1. Biochemical and physiological characteristics of wild and mutant yeast strain

| In the Table below, (+ indicates the presence of activity for the specified test, - indicates the absence of activity for the specified test). | | |
| --- | --- | --- |
| **Test** | **Wild type** | **Mutant** |
| **Growth on** | | |
| L-Sorbose | - | + |
| D-Ribose | - | - |
| D-Xylose | + | + |
| Sucrose | + | + |
| Cellobiose | + | + |

(continued)

In the Table below, (+ indicates the presence of activity for the specified test, - indicates the absence of activity for the specified test).

| Test | Wild type | Mutant |
|---|---|---|
| **Growth on** | | |
| Salicin | + | + |
| Raffinose | - | - |
| Inulin | - | - |
| Starch | + | - |
| Citrate | - | - |
| Methanol | - | - |
| Ethanol | + | - |
| Sulfur (5 ppm) | + | + |
| Sulfur (50 ppm) | - | + |
| Sulfur (400 ppm) | - | + |
| Sulfur (1000 ppm) | + | + |
| Tridecane (1%) | + | + |
| Tetradecane (1%) | + | + |
| Dodecane (1%) | + | + |
| Hexadecane (1%) | + | + |
| Hexadecane (25%) | + | + |
| Adipic acid (0.2%) | + | + |
| Glutaric acid (0.2%) | + | + |
| Pimelic acid (0.2%) | + | + |
| Dodecanoic acid (0.2%) | - | + |
| CeO2 (10 ppm) | - | + |
| **Growth temperature** | | |
| 25°C | + | + |
| 37°C | + | + |
| 42°C | + | + |
| 45°C | - | + |
| 50°C | - | - |
| **Enzyme activity** | | |
| Cyt-P450 enzyme concentration | 3.58 $\mu$mol/ml | 12.25 $\mu$mol/ml |
| **Fermentation of** | | |
| Kerosene | - | + |
| Tetradecane | + | + |
| Dodecane | + | + |
| Hexadecane | + | + |
| Physiological properties | | |

(continued)

| Fermentation of | | |
|---|---|---|
| Budding cells | - | + |
| Splitting cells | + | + |
| Filamentous | - | - |
| Pink colonies | - | + |

**Example 2: Evaluating the concentration of Cytochrome-p450 in mutant yeast strains**

In order to evaluate the concentration of cytochrome-p450 in mutant yeast strains, the cells were added to 1mL of leukocyte lysis buffer (WCLB) consisting of 10 mM Tris (pH 7.6), 10 mM EDTA (pH 8.0), 50 mM NaCl, 0.2% sodium dodecyl sulfate (SDS), and 200 mg of proteinase K per mL, and incubated at 65°C for 1hour. After which cells were pelleted for 5 minutes at 5000 g, and 10mL of the supernatant sample was used for the analysis of Cytochrome-p450 using the following protocol as described by Guengerich et.al Nature protocols (2009): 1245-1251.

Briefly, the sample was transferred into 2.0ml of 100 mM of potassium phosphate buffer (pH 7.4-7.7) containing 1.0 mM of EDTA, 20% glycerol (vol/vol), 0.5% sodium cholate (wt/vol) and 0.4% (wt/vol) non-ionic detergent (e.g., Triton N-101) in a small test tube. The sample was mixed by capping the test tube with parafilm and inverting/re-inverting several times. The sample was divided into two 1ml cuvettes, using a glass pasteur pipette and a bulb. The two cuvettes were placed in the spectrophotometer and record a baseline between 400 and 500 nm. The sample was removed from cuvette from the spectrophotometer and (in the fume hood) slowly a CO gas was bubbled through this sample from a clean pasteur pipette. The rate should be about one bubble per second. ~1 mg of solid $Na_2S_2O_4$ and dithionite was added to both cuvettes and spectra was measured between 400 and 500 nm several times, over a period of a few minutes, till the size of the peak near 450 nm has stopped increasing. The absorbance was taken at 450 and 490 nm.

The concentration of cytochrome P450 was calculated by the following formula:

$$(\triangle_{A450} - \triangle_{A490})/0.091 = \text{nmol of Cytochrome-p450 per ml.}$$

The concentration of Cytochrome-p450 in wild strain was found to be 3.58 $\mu$mol/ml where as in mutants it was reported as 12.25 $\mu$mol/ml.

**Example 3: Bioassisted process for conversion of alkane (Kerosene) using mutant strain *Candida tropicalis* (Castellani) Berkhout (ATCC 750)**

Initially, enriched mutant of *Candida tropicalis* (Castellani) Berkhout (ATCC 750) was selected and grown overnight in 2% malt media at 37 °C with rotation at 200rpm. In a separate process, iron- doped $CeO_2$ was prepared by the following procedure: Typically, 0.02 M solution of Ce (III) nitrate was prepared in 250mL distilled water followed by addition of 0.005 M $FeCl_2$.

Similarly, 0.03 M of $K_2CO_3$ solution was prepared by dissolving required amount of $K_2CO_3$ in 250 mL of distilled water. An aqueous solution of iron-containing Ce (III) nitrate (50 mL) and potassium carbonate (20 mL) was added drop by drop to a well-stirred water (100 mL) to precipitate a white-brown precursor, namely iron doped cerium (III) carbonate. The neutral pH was maintained during the precipitation method. Resulting iron doped $CeO_2$ were dried at 65∘C for 2hours, cooled to room temperature. Then, the product was aged at 220°C for 2.5 hours without any washing and purification and finally calcined at 600°C for 3 hours. Further, to 250 ml of Luria-Bertani (LB) medium, 2g of iron-doped $CeO_2$ was added followed by addition of 0.5 g of the centrifuged wet microbial cell. To the same medium, 50 ppm of polyoxyethylene nonyl phenyl ether was added dropwise. The culture medium was inoculated at 37°C for 2 days.

Subsequently, the conversion/biosynthesis was conducted in a 2litre reactor at 30°C, 200 rpm. 1g wet weight of the cells obtained was washed 3 times in phosphate buffer and transferred to a 1L media containing 0.5% Tween 20, 1X filter sterilized trace element solution (g/L: 2.4 g of $FeCl_3·6H_2O$, 0.3 g of $CoCl_2·H_2O$, 0.15g of $CuCl_2·2H_2O$, 0.3 g of $ZnCl_2$, 0.3g of $Na_2MO_4·2H2O$, 0.075 g of $H_3BO_3$, and 0.495 g of $MnCl_2·4H_2O$. After the OD reached 2-3, 25% kerosene having sulfur content 1250 ppm was added to it. Different concentration of oxygen (1% to 100 %, balance N2) was sparged to the reactor with 2 ml/min. The samples were collected at different time points for product quantification and identification by gas chromatography (GC).

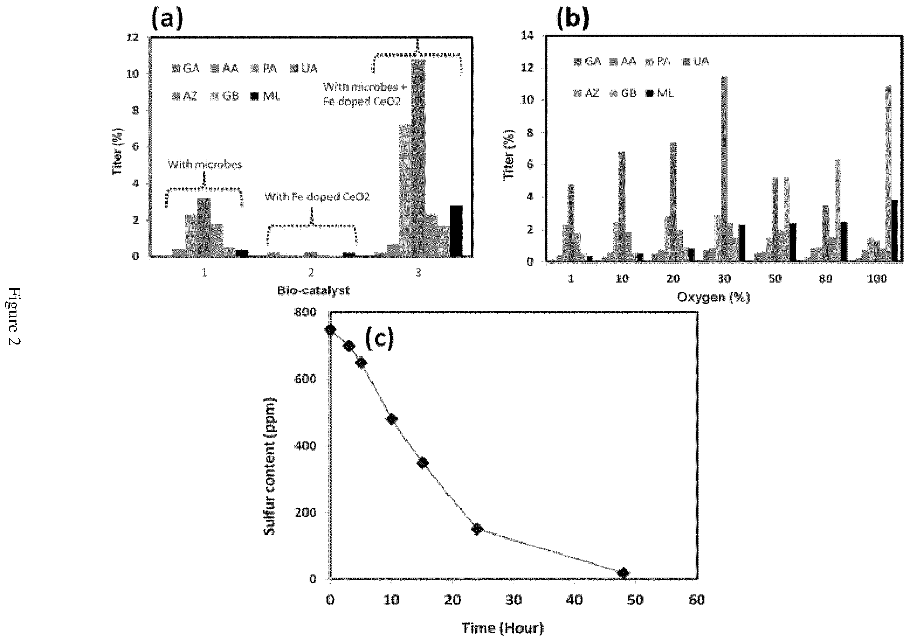

The Figure 2 as stated above, represents the product concentration in the presence of mutant yeast strain, neat iron doped $CeO_2$ and microbe hybridized $Fe/CeO_2$. It has been found that the titer value significantly increases in microbe hybridized iron doped $CeO_2$. The increase in product formation clearly implies that the synergistic effect of microbe hybridized iron doped $CeO_2$ facilitates the feed utilization by increasing the metabolism of yeast. Further, it was reported that the selectivity increases with increase in the oxygen concentration. With $O_2$ concentration 30%, the selectivity of undecanoic acid is -90% whereas, with an $O_2$ concentration of 100 %, the glycolipid biosurfactants reaches around 90%. Moreover, it was found that the total initial sulfur content (~800 ppm) in the feedstock drastically reduced to -51ppm in 48 hours of biotransformation. This clearly indicates that yeast strains have the ability to desulfurize the feedstock along with the product formation.

**Claims**

1. A bio-assisted process for selective conversion of alkane rich refinery stream, the process comprising:

    (i) growing a mutant yeast strain in 2% malt media at a temperature of 37°C with rotation at 200rpm till OD of the media reaches 2-4;
    (ii) centrifuging the media of step (i) to obtain wet microbial cells;
    (iii)adding the centrifuged wet microbial cells of step (ii) to a heterogenous nanocatalyst in a culture media, followed by intermittent feeding of a stabilizer in concentration ranging from 50-100 ppm;
    (iv)incubating the media of step (iii) with 1-25% stream in a reactor at a temperature ranging from 35-37°C for a duration of 1-2hours;
    (v) sparging oxygen gas to the reactor to obtain a product, wherein the product is selected from mono and dicarboxylic acid, fatty acids and biosurfactants.

2. The process as claimed in claim 1, wherein the mutant yeast strain is selected from the group consisting of *Candida vini*, *Candida entamophila, Candida blankii* and *Pichia Farinosa, Candida tropicalis (Castellani) Berkhout* (ATCC 750).

3. The process as claimed in claim 1, wherein the mutant yeast strain is *Candida tropicalis* (Castellani) Berkhout (ATCC 750).

4. The process as claimed in claim 1, wherein the obtained product is a dicarboxylic acid.

5. The process as claimed in claim 4, wherein dicarboxylic acid is undecanoic acid.

6. The process as claimed in claim 1, wherein the alkane rich refinery stream comprises sulphur in the range of 5 ppm to 25000 ppm.

7. The process as claimed in claim 1, wherein the alkane rich refinery stream comprises hydrocarbons having carbon atoms ranging from 6 to 16.

8. The process as claimed in claim 1, wherein the oxygen gas sparged in the reactor contains 20-100% $O_2$ along with nitrogen and atmospheric air.

9. The process as claimed in claim 1, wherein the heterogenous nano catalyst comprises:

    (i) oxides of molybdenum, vanadium, antimony, niobium;
    (ii) at least one element selected from the group consisting of lithium, titanium, tin, germanium, zirconium, hafnium; and
    (iii) optionally at least one lanthanide selected from the group consisting of lanthanum, praseodymium, neodymium, samarium, europium, gadolinium, dysprosium, holmium, erbium, thulium, ytterbium and lutetium.

10. The process as claimed in claims 1-9, wherein the heterogenous nano catalyst is iron doped cerium (III) carbonate.

11. The process as claimed in claim 1, wherein the stabilizer is selected from long chain alkylamine or N-based alkylamines and derivatives, N-[2-[(2-Aminoethyl) amino] ethyl]-9-octadecenamide, n-Benzalkonium chloride (BAC), CnH(2n+1)-COO(CH2CH2O)12CH3, polyoxyethylene alkyl ether, n-alkyl trimethyl ammonium surfactant, potassium alkanoate, dodecylpyridinium bromide, octylglucoside, Sodium dodecyl sulfate, trans-Cinnamaldehyde, Sodium bis-(2-ethylhexyl)-sulfosuccinate, cetylpyridinium chloride, Primary alcohol ethoxylate, polyoxyethylene nonyl phenyl ether, polyethylene glycol esters, linoleate and dodecyl amine.

12. The process as claimed in claim 1, wherein the obtained product is separated by column chromatography and unreacted alkane is circulated back to reactor for fermentation.

13. A mutant yeast strain for selective conversion of alkane rich refinery stream from a substrate containing hydrocarbons selected from the group consisting of *Candida vini*, *Candida entamophila, Candida blankii* and *Pichia Farinosa, Candida tropicalis (Castellani) Berkhout.*

Figure 1

Figure 2

Europäisches
Patentamt

European
Patent Office

Office européen
des brevets

# EUROPEAN SEARCH REPORT

Application Number

EP 22 19 6092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2010/068904 A2 (DU PONT [US]; RITTER JOACHIM C [US] ET AL.) 17 June 2010 (2010-06-17) * page 2, lines 18-23 * * page 3, line 8 - page 5, line 28 * * page 12, lines 1-21 * * page 19, line 10 - page 21, line 13 * * page 23, line 6 - page 25, line 5; examples 1-13 * * claims 1-17 * | 1-13 | INV. C12P7/46 C12P7/6409 C12P7/44 C12N15/01 C12R1/74 |
| A | US 2010/196973 A1 (DUBOIS JEAN-LUC [FR]) 5 August 2010 (2010-08-05) * page 3, paragraph [0020] - page 4, paragraph [0029]; examples 1-5 * * claims 1-8 * | 1-13 | |
| A | BARDANIA HASSAN ET AL: "Investigation of Desulfurization Activity, Reusability, and Viability of Magnetite Coated Bacterial Cells", IRANIAN JOURNAL OF BIOTECHNOLOGY, vol. 17, no. 2, 1 June 2019 (2019-06-01), pages 14-20, XP093021558, DOI: 10.21859/ijb.2108 * abstract * * page 15, left-hand column, paragraph 4 - page 16, left-hand column, paragraph 1; figure 3 * | 1-12 | TECHNICAL FIELDS SEARCHED (IPC) C12P C12R C12N |
| X | US 3 622 455 A (IIZUKA HIROSHI ET AL) 23 November 1971 (1971-11-23) * column 1, lines 5-73 * | 13 | |

-/--

The present search report has been drawn up for all claims

| Place of search | Date of completion of the search | Examiner |
|---|---|---|
| Berlin | 7 February 2023 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 6092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SAHOO PRAKASH C. ET AL: "Photosensitized biohybrid for terminal oxygenation of n-alkane to [alpha], [omega]-dicarboxylic acids", MOLECULAR CATALYSIS, vol. 535, 1 January 2023 (2023-01-01), page 112889, XP093021389, ISSN: 2468-8231, DOI: 10.1016/j.mcat.2022.112889 * the whole document * | | |

TECHNICAL FIELDS
SEARCHED       (IPC)

The present search report has been drawn up for all claims

| Place of search | Date of completion of the search | Examiner |
|---|---|---|
| Berlin | 7 February 2023 | Mateo Rosell, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 19 6092

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2023

| Patent document cited in search report | | Publication date | Patent family member(s) | | Publication date |
|---|---|---|---|---|---|
| WO 2010068904 | A2 | 17-06-2010 | CN | 102439118 A | 02-05-2012 |
| | | | EP | 2356198 A2 | 17-08-2011 |
| | | | HK | 1170256 A1 | 22-02-2013 |
| | | | JP | 5726088 B2 | 27-05-2015 |
| | | | JP | 2012511907 A | 31-05-2012 |
| | | | KR | 20110101198 A | 15-09-2011 |
| | | | US | 2011300594 A1 | 08-12-2011 |
| | | | WO | 2010068904 A2 | 17-06-2010 |
| US 2010196973 | A1 | 05-08-2010 | BR | PI0816431 A2 | 14-10-2014 |
| | | | CN | 101868552 A | 20-10-2010 |
| | | | EP | 2191003 A1 | 02-06-2010 |
| | | | FR | 2921363 A1 | 27-03-2009 |
| | | | JP | 6073044 B2 | 01-02-2017 |
| | | | JP | 2010539225 A | 16-12-2010 |
| | | | US | 2010196973 A1 | 05-08-2010 |
| | | | WO | 2009047444 A1 | 16-04-2009 |
| US 3622455 | A | 23-11-1971 | BE | 724553 A | 02-05-1969 |
| | | | DE | 1812710 A1 | 03-07-1969 |
| | | | FR | 1595297 A | 08-06-1970 |
| | | | GB | 1218476 A | 06-01-1971 |
| | | | US | 3622455 A | 23-11-1971 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006064131 A1
- CN 102191187 A
- US 20040146999 A1
- US 20130267012 A1
- WO 2007077568 A1
- WO 2010068904 A2
- US 10604775 B2
- US 8383373 B2

**Non-patent literature cited in the description**

- **KITAMOTO.** *Biotechnology Letters,* 2001, vol. 23, 1709-1714
- **GUENGERICH.** *Nature protocols,* 2009, 1245-1251